# EUROPEAN PATENT APPLICATION

(11) **EP 1 417 985 A1**
(43) Date of publication of application: **12.05.2004**
(21) Application number: 03024504.7
(22) Date of filing: 24.10.2003
(51) Int. Cl.: A61N 1/36

(54) **Electrical stimulation device particularly for neuromuscular stimulation**

(30) Priority: 08.11.2002 IT TV20020134
(71) Applicant: Globus Italia S.r.l., 31013 Codogne' (Prov. of Treviso) (IT)
(72) Inventor: Bertocco, Rossano, 30030 Oriago Di Mira (Venezia) (IT); Lucchetta, Pierpaolo, 31020 San Vendemiano (Treviso) (IT)
(74) Representative: Modiano, Guido, Dr.-Ing.

(57) **Abstract**

An electrical stimulation device provided with a memory (2, 3) that comprises sequences of data related to electrical pulses to be generated, at least one electrical terminal (6), and control elements (1) for generating, adjusting and transmitting electrical pulses on the at least one electrical terminal according to the data sequences stored in the memory; the device further comprising means (9) for writing, in the memory (2, 3), information regarding the electrical pulses transmitted to the at least one electrical terminal, the information being settable by a user.

## Description

The present invention relates to an electrical stimulation device particularly useful for neuromuscular stimulation for therapeutic purposes and for improving and maintaining muscle tone.

Electrical stimulation devices are widely used both in the physiotherapy field and among private users and are typically used to replace massages or physical activity in a gymnasium. It is well-known that they are constituted by electronic control means that are connected to a plurality of electrical terminals to be applied to the body, for example by means of suction cups, belts, bands, adhesive electrodes or other devices suitable to apply electrical current to a human body in order to stimulate, by means of electrical pulses, the contraction of the muscles to which said electrodes are attached; each electrical terminal is normally constituted by a pair of electrodes.

The electrical pulses are controlled in terms of intensity, frequency and duration by the control means and constitute the basic elements with which electrical stimulation sessions are designed.

The distribution of the pulses and their organization within an electrical stimulation session can be provided in various manners.

One particular organization is, for example, of the hierarchical type: an electrical stimulation session, in this case, comprises the execution of one or more work programs. A work program is divided into a certain number of steps, characterized by the repetition of stimulation cycles in which the intensity, frequency and duration of the electrical pulses is adjusted so as to achieve contraction and relaxation of the muscle. In further detail, the stimulation cycle is divided into segments: the muscle is subjected to a more demanding work segment and to a rest segment that is instead characterized by a more bland stimulation level. The work segment is mainly composed of an effective segment, in which a certain value of current is maintained and in which the stimulation can be increased or decreased by the user, for example by means of a keypad and a display connected to the control means. Optionally, the work segment can include a rising ramp and a falling ramp, in which the stimulation is respectively increased to the value defined in the effective segment or decreased to zero.

A program typically comprises an initial warm-up step, which has a medium pulse repetition frequency and a duration that can vary between five and ten minutes, an intermediate work step, having a high pulse repetition frequency and a duration between ten and typically thirty minutes, and a final winding-down step, in which the repetition frequency is low and the duration is comprised between five and ten minutes. One known work step, for example, uses two-phase pulses lasting 200 microseconds at the frequency of 100 pulses per second.

The control means are typically constituted by a microprocessor or microcontroller, to which memory means are connected which comprise sequences of data related to the electrical pulses to be generated. These data sequences are organized in records, which contain all the information for generating the pulse and controlling the cycle, the phases and the stimulation program.

Known electrical stimulation devices are completed by a power supply or DC/DC converter, devices for interfacing with the user, and a voltage pulse source for each pair of electrodes.

By means of the user interface devices, such as for example a keypad and a display, it is possible to select and start a program and adjust the amplitude of the delivered current according to one's own perceptions or requirements.

In the standard starting sequence of an electrical stimulation program, the initial current is set to zero and the user increases it until a chosen value is reached. In the subsequent steps, the stimulation current set at the beginning of each new work step is calculated according to specific formulas and is produced and finally regulated by the user for final adjustments.

From what is apparent from the prior art, the user adjusts the intensity of the pulses generated according to his own perceptions or requirements and is forced to repeat, at each session, the same sequence or a very similar sequence of adjustment operations performed in the preceding session or during a preceding work step or program. Accordingly, if a program contains a plurality of work steps, the user has to adjust the intensity of the pulses again according to the same previous criterion for each work step, i.e., by following his own perceptions or possibly by following the instructions of a manual.

The repetition of the sequence of adjustment operations entailed by each activation of a same program (or during the execution of the program) can be tedious for the user or even complicated or impossible when the user is reluctant with respect to electronic devices in general.

Repeating the same operations can also become a problem for physiotherapists who have to adjust simultaneously a plurality of machines connected to different patients, taking into account all the particular operations performed for each patient.

The aim of the present invention is to eliminate the drawbacks described, by providing an electrical stimulation device and a method for its management that allow to simplify the periodic use of the electrical stimulation device, avoiding repetitive interventions on the part of the user in order to adjust the output characteristics of the device.

Within this aim, an object of the present invention is to minimize user adjustment interventions that are not related to his personal perceptions as a consequence of the activation of a particular program.

Another object of the invention is to avoid limiting user interventions in any case, retaining the freedom to modify the values that characterize the stimulation at any time.

This aim and these and other objects that will become better apparent hereinafter are achieved by an electrical stimulation device according to the invention, comprising:
memory means that comprise sequences of data related to electrical pulses to be generated;
at least one electrical terminal;
control means for generating, adjusting and transmitting electrical pulses on said at least one electrical terminal according to said data sequences stored in the memory means;
characterized in that it comprises means for writing, in the memory means, information regarding the electrical pulses transmitted to said at least one electrical terminal, said information being settable by the user.

Further characteristics and advantages of the present invention will become better apparent from the following detailed description of a preferred but not exclusive embodiment of the electrical stimulation device, illustrated by way of nonlimitative example in the accompanying drawings:
Figure 1 is a block diagram of the hardware structure of the electrical stimulation device according to a known embodiment;
Figure 2 is a schematic view of the first memory (Program memory) and of the second rewritable memory, which is divided into the Last10 memory and the Favorites memory;
Figure 3 is a flow chart of the procedure for selecting the program from the memories according to the invention;
Figure 4 is a flow chart of the procedure for functional management of the automatic operating mode according to the invention;
Figure 5 is a flow chart of the management procedure of standard operation according to the invention;
Figure 6 plots the intensity of the output stimulation as a function of time at the start of a program in automatic mode.

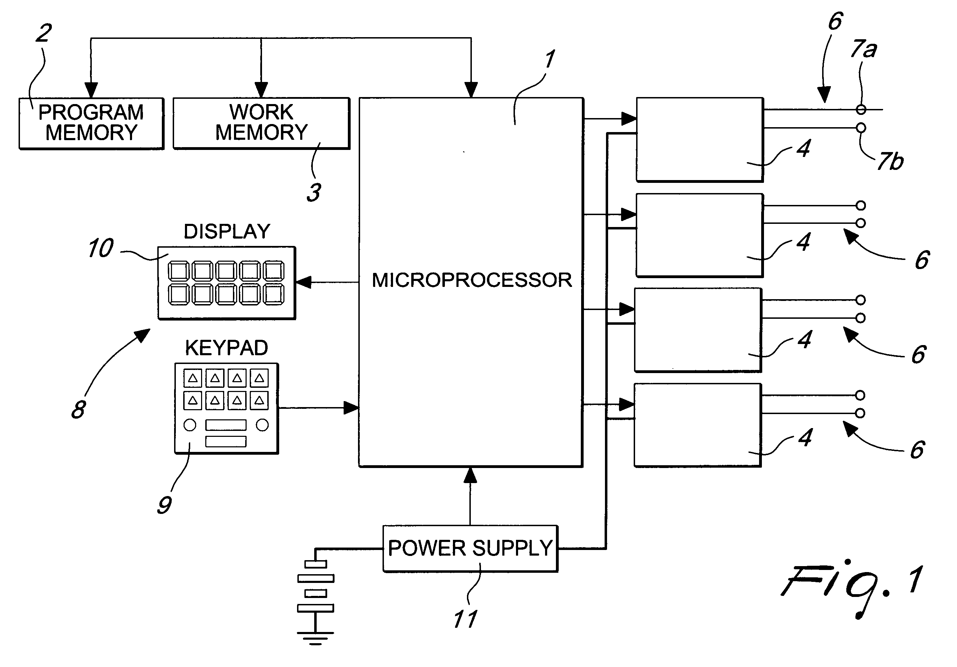

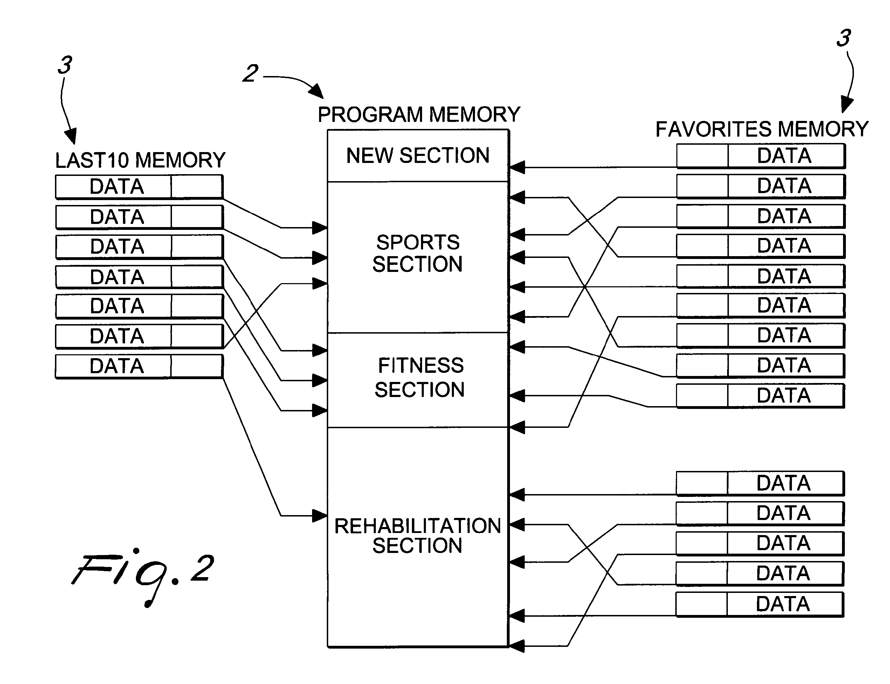

With reference to Figures 1 and 2, a preferred structure for an electrical stimulation device with a plurality of outputs comprises a microprocessor 1 connected to memory means.

The memory means comprise the information required to generate the pulses and to control the programs and comprise information related to the electrical pulses transmitted during the particular program being executed; the information regarding the transmitted electrical pulses is stored appropriately as will be described hereinafter.

Preferably, the memory means comprise two permanent memory areas or banks 2 and 3, for example of the EEPROM type. The first memory bank 2, designated hereinafter as program memory, is preferably accessible at read-only level and comprises the information records required to generate the pulses and control the cycle, the steps and the programs.

The program memory 2 can be accessed also in write mode in the upper part of the memory, so that a qualified person can create new programs or new work steps.

The second memory bank 3, designated hereinafter as work memory, comprises the references to the programs stored in the program memory 2 and information related to the electrical pulses transmitted as a consequence of the execution of a program. The work memory 3 is accessible in read mode and in write mode and is used to memorize the way in which the particular loaded program is executed, so as to provide additional data if the program is restarted in a subsequent work session.

The memory means are not necessarily constituted by two memory banks but can comprise a single bank or more than two banks according to the requirements.

With reference to Figure 2, the work memory 3 is preferably organized into several logic subsections. In the preferred embodiment, a first subsection, designated by the caption Last10, comprises the references and data related to the last ten programs executed; a second subsection, designated Favorites, instead comprises the references and data related to a certain number of programs that are used frequently.

The work memory 3 can comprise additional subsections, not shown in Figure 2, such as for example specific user areas, in which customized information related to the users of the electrical stimulation device is stored.

As shown in the figure, both the Last 10 section and the Favorites section point to particular areas of the program memory 2, in that they contain cells for addressing a particular record of the program memory 2.

The information related to the transmitted electrical pulses is referenced hereafter also as work values, i.e., values linked to the intensity of the stimulation applied to the muscle and to the methods of use and execution of the part of the user.

Stimulation intensity depends, for example, on the intensity of the electrical pulses applied to the muscle or on the frequency of repetition of the electrical pulses or on their modulation or on a combination of these or other parameters.

The program memory 2 also can be divided logically into a plurality of separate sections, each of which contains equivalent programs: for example, Figure 2 shows, in addition to the upper section designated by New, which can be customized by an expert user, a Sports section, a Fitness section and a Rehabilitation section.

The microprocessor 1 comprises a temporary memory, for example RAM, in order to temporarily store information related to the program in execution and the work values stored during the execution of the program.

The work values are stored automatically during the execution of a program, as will become better apparent hereinafter.

Moreover, the microprocessor has a plurality of outputs that connect it to a series of power modules 4 in order to generate electrical pulses; an electrical terminal 6, constituted by two electrodes 7a and 7b, protrudes from each one of the power modules 4.

The microprocessor is further connected to devices 8 for interfacing with the user, such as a keypad 9 and a display 10, and comprises a timer (not shown in the figures), which counts the duration of a work step of the particular program that is loaded.

Typically, the specifications require the application of a current of ± 120 mA on a load of 1000 ohm, and for this purpose it is necessary to provide the pulse generator with a voltage of at least 150 V, which can be obtained for example from a power supply which is connected to a 7.2 V DC battery at 1300 mA/h.

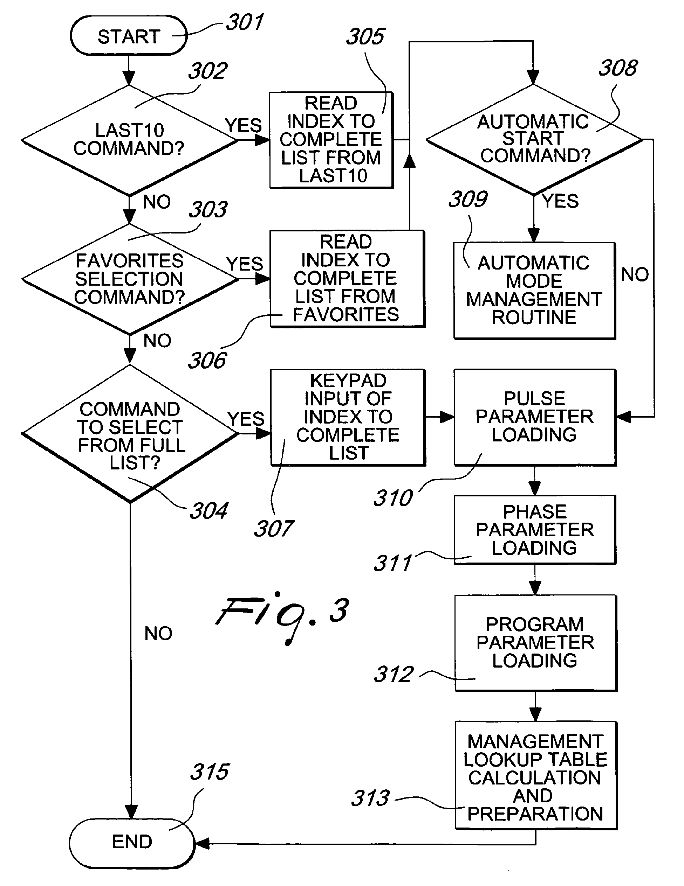

The operation of the electrical stimulation device follows the steps shown schematically in Figures 3 to 5.

In particular, Figure 3 illustrates the flow chart of the procedure or routine for selecting the program from the memory means according to the invention. Differently from known devices, the operation of the electrical stimulation device according to the invention comprises the steps 308 and 309, by means of which it is possible to start and manage a kind of operation that is termed automatic; automatic operation is characterized in that it comprises steps for storing and/or loading into memory work values set by the user.

In the preferred embodiment described hereinafter, the values are set by the user according his own perceptions during the execution of a work program; the values are analyzed and processed by the software and then stored automatically. In this manner, the non-expert user can set the values without knowing the electrical details of the pulses that are transmitted to the stimulated muscle region.

Moreover, the work values can be set and stored by means of a specific command or at the user's request. In this case also, the stored work values are in any case proposed again and applied when the same program is restarted.

The operation of the program selection procedure of the particular embodiment shown in the figures consists in verifying, after the steps for power-on and initialization of the components that precede the start 301 of the program selection procedure, the user's activation of the program selection commands by means of the index contained in the work memory and termed Last10 (step 302) or by means of the indeed termed Favorites (step 303). The user can then activate a program rapidly when it has been used recently or when it is in any case used frequently.

Moreover, the user can load a program by scrolling through a complete list of programs; from the point of view of the procedure shown in Figure 3, if both tests in steps 302 and 303 have a negative outcome, the command for selection from the complete program list is activated in step 304. If this test also has a negative outcome, the program selection procedure is abandoned in step 315 in order to move on to subsequent steps, such as testing of the power-off command or testing of the command to program the high level of the program memory (steps not shown in the figures); in any case, from step 315 the system stands by for keypad commands.

If the test in step 304 instead has a positive outcome, after a step 307 for reading the index of the complete program list on the part of the microprocessor and for consequently selecting the chosen program on the part of the user, the parameters of the pulses, of the work steps and of the program are loaded respectively in steps 310, 311, 312, and the lookup tables are calculated and prepared only at the end. The lookup tables are then scanned by a timer function in order to extract the appropriate parameters for activating the outputs, connected to the body of the user, of the electrical stimulation device during functional operation.

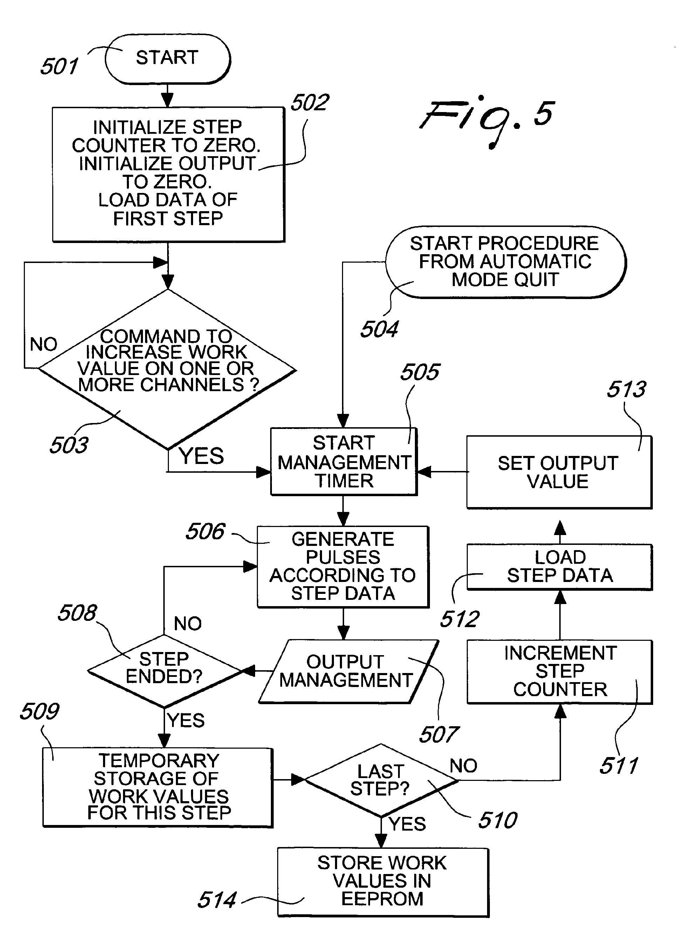

When leaving the program selection procedure, the system enters the functional operating mode known as Standard or Manual, shown in Figure 5; the details of the management procedure are described hereafter.

In step 302 or 303, if the test has a positive outcome, the indexes to the Last10 or Favorites list are read respectively in step 305 or in step 306; regardless of the originating block, the system then moves on to step 308, in which the state of activation on the part of the user of the automatic operating mode, described hereafter, is checked. In other words, the system stands by for user confirmation of the intention to activate the automatic operating mode. However, the block 308 is optional, in that once a particular program has been selected, the system can activate directly the automatic operating mode without requesting confirmation. As will become better apparent hereinafter, the user can in fact in any case quit the automatic operating mode and move to the standard operating mode, for example when the perceived stimulation intensities do not correspond to the actual requirements.

If the test in step 308 has a negative outcome, the system moves on to step 310 and proceeds as before, using the standard work parameters of the particular selected program; otherwise, in step 309 the information related to the transmitted pulses, stored automatically at the end of the execution of the preceding work session in the work memory 3, are loaded into the temporary memory. The information related to the transmitted pulses (or work values) stored automatically in the work memory 3 is preferably loaded after checking, again in step 309, that these values actually exist in memory.

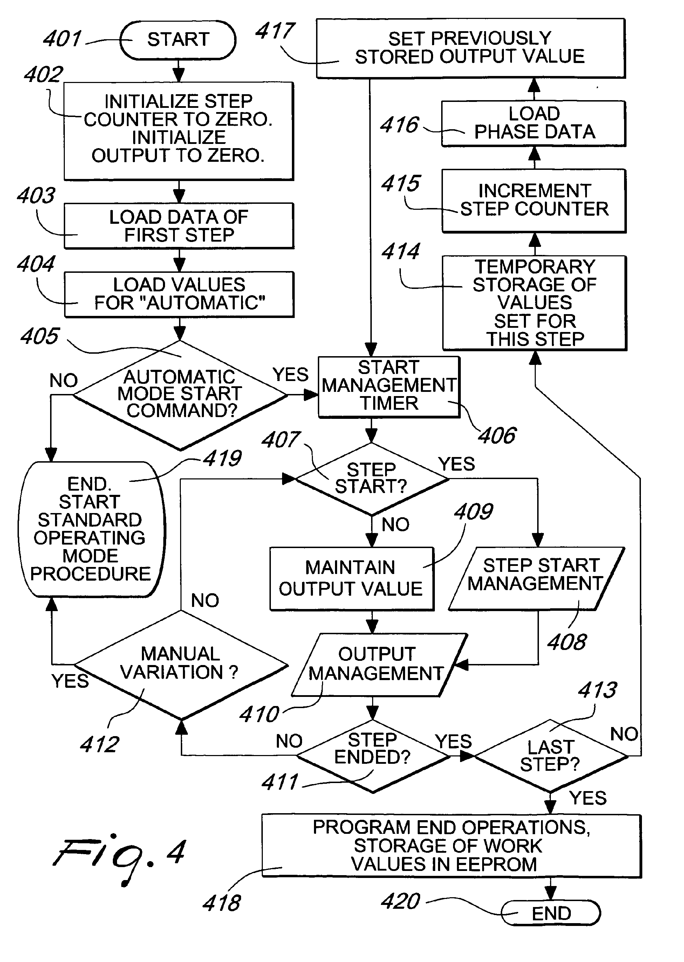

Automatic storage is one of the particular characteristics of the invention, and the corresponding procedure will be described hereinafter with reference to Figures 4 and 5.

With reference to Figure 4, after the start 401 of the procedure for functional management of the automatic mode, step 402 initializes a counter of the work step that is internal to the program selected by the user, and the output current is set to zero. The work step counter is used to identify the active step of the program being executed.

The initialization step 402 is followed by a first step 403 for read access to the memory means via said control means, in which data related to the first work step contained in the selected program are loaded, and by a step 404, in which the work values stored in a preceding work session are loaded.

If the user, in step 405, does not confirm the automatic operation mode, the procedure is interrupted at step 419 and the electrical stimulation device assumes the standard functional operating mode, described hereafter; in the particular embodiment shown, the user does not confirm the automatic operating mode if he increases manually the current to be sent to the electrodes, i.e., if he proceeds as in known embodiments. In known embodiments, electrical stimulation is in fact started when a signal to increase the current on the part of the user arrives.

If the automatic operating mode is confirmed in step 405, in step 406 the management timer is started and counts the duration of a work step, and in step 407 the system is checked to determine whether it is at the beginning of the current work step, i.e., of the work step identified by the step counter; if the outcome of this test is positive, the system reaches the state 408, in which a procedure for automatic variation of stimulation intensity is started.

In the preferred embodiment, the procedure 408 consists of a soft start that comprises an attenuation step followed by a step for gradually restoring the amplitude values stored in the preceding work session.

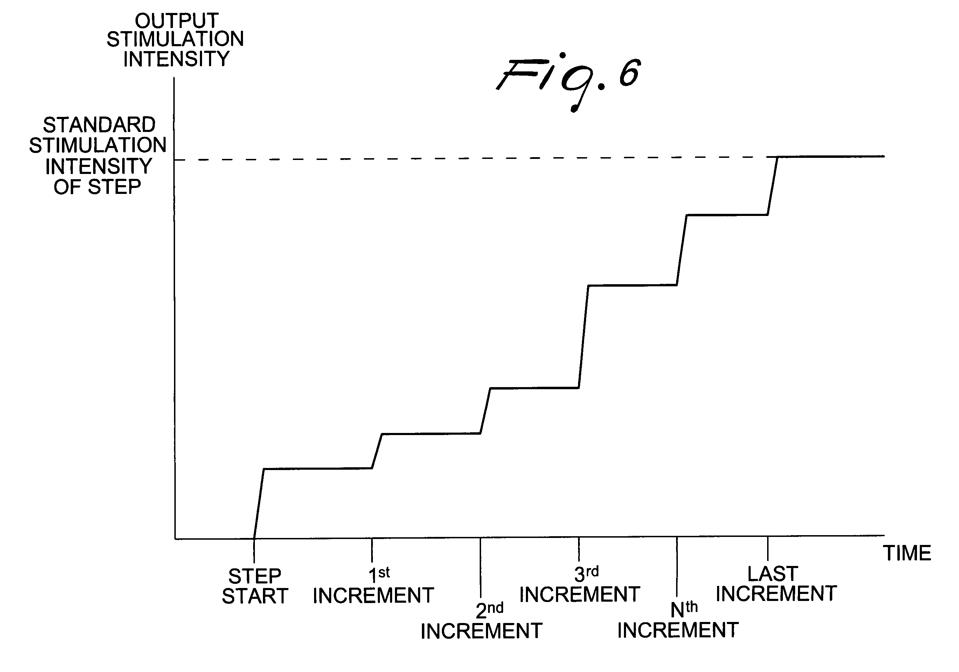

In the soft start procedure in particular, a fraction (for example 10%) of the current stored in the preceding work session is set initially, and pulses are produced whose amplitude gradually reaches 100% over a few stimulation cycles (for example ten): stimulation intensity over time can be constituted by a linear variation or by a discrete stepwise variation, as shown by way of example in Figure 6.

Each program contains, among its various items of information, a table with the data required in order to define the variation of the generated current, particularly the percentage variation to be applied to the amplitude of the current stored and the time interval required in order to perform this increase. Once the entire table has been scanned and therefore 100% of the previously stored current has been reached, the program proceeds by keeping constant the output current up to the end of the work step.

The soft start procedure is meant to "soften" the start of a stimulation step, at the same time eliminating effects of habituation to the electrical stimulation.

The variation performed in step 408, however, is not limited only to restoring the work values stored in a preceding work session, but can be different according to the applications. The stimulation intensities reached during or at the end of the step 408 can in fact even exceed 100% of the previously stored values.

Alongside the step start procedure 408, in step 410 a procedure for managing the output current ensures that the current supplied to the user corresponds to the current actually displayed on the display.

If the test of step 407 has a negative outcome, i.e., if the system is not at the beginning of a work step, in the block 409 the stimulation intensity is kept constant; at the same time, the procedure for managing the output current is performed in step 410.

Step 411 checks whether the time set by the timer has expired, i.e. whether the current work step has ended; if it has not, an (optional) step is entered which further checks whether in the meantime the user has set manually the value of the current (step 412). If manual setting has been performed, the operating management procedure of the automatic operating mode is terminated and the system enters the standard operating mode (step 419).

If the work step has not ended and the user has not manually changed the current values during said work step, the device resumes operation starting from step 409, as before, maintaining the current value.

If instead the work step of the program has ended, step 413 checks whether the step that has just been performed is the last step of the program selected by the user; if it is, program end operations are performed in step 418, and in particular the work values stored in the temporary memory during program execution are stored in the work memory 3.

Step 418 is followed by exit 420 from the automatic mode operating management procedure and the electrical stimulation device enters standby (not shown in the figures), awaiting new commands.

If the step that has just ended is not the last of the series of work steps that constitute the program, the work value (for example the intensity of the current) reached by the system is stored in the temporary memory. In particular, the previously read work value is confirmed.

Temporary storage of the work values during the execution of the automatic operating mode management procedure can be performed during said work step and not necessarily at the end of the step, as described previously by way of example.

After increasing in step 415 the counter that indicates the new work step, the procedure loads into memory the data related to the new work step (step 416) and sets the output current to the work value stored previously for the new work step.

Then the procedure restarts the timer and the cycle is repeated as described earlier.

Differently from the standard procedures used in known electrical stimulation devices, loading of the work values stored in a preceding work session and storage of said work values are both provided.

In particular, as anticipated earlier, if the user does not select the automatic operating mode or manually varies the current within a work step, the standard operating management procedure is activated; said procedure is advantageously modified and improved with respect to known procedures. The improvement consists indeed in storing the work values of the various executed work steps; the work values can be set manually by the user during the execution of the work program. If there are no user interventions, the work values (loaded previously) of the particular step in execution are overwritten.

At the same time, the standard operating management procedure allows to resume the sequence of operations previously set by the procedure shown in Figure 4.

Figure 5 is the flow chart of the standard operating management procedure mentioned earlier. After the common steps of startup, initialization and loading of the data related to the first step (steps 501 and 502), pulse generation is activated by a command provided by the user via the keypad:
the command, in the particular embodiment indicated in step 503, consists for example of a command to increase the work value on one or more output channels.

This type of command is similar to the one used in known embodiments, in which the pulses are applied when the user increases the intensity of the current on one or more channels.

As a consequence of the command of step 503, the management timer defined earlier is started in step 505. Step 505 can also be reached via the startup step 504, which the system reaches when the automatic operating procedure is abandoned, as described earlier.

After the steps for pulse creation, output current management and control of work step termination (506, 507 and 508), similar to steps 409, 410 and 411 described earlier with reference to Figure 4, at the end of the work step the work value used in the step that has just ended is stored in the temporary memory.

The standard operation management procedure then continues according to the same known steps: checking the step counter in order to test whether the work step that has ended is the last step of the program (step 510), incrementing the step counter in step 511 if the test 510 has a negative outcome, and loading the data related to the work step that follows the step that has just ended (step 512).

The output current values loaded in step 512 are set in step 513 and suitable correction coefficients Ke and Kr are optionally applied.

The cycle is then repeated starting from step 505. In the steps designated by the reference numeral 514, which the system reaches after executing the last work step of the program, the operations for closing the program and storing in the work memory 3 the work values stored temporarily in the RAM memory in step 509 are performed.

Automatic storage of the set current values and their resubmission in subsequent work sessions are not mandatory for the user of the electrical stimulation device according to the invention. Saving in the work memory is performed in any case, in the particular embodiment described; when a particular program is repeated, the previously set work values are loaded if the user selects the automatic operation mode.

The invention thus conceived is susceptible of numerous modifications and variations, all of which are within the scope of the appended claims. All the details may further be replaced with other technically equivalent elements.

The disclosures in Italian Patent Application No. TV2002A000134 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. An electrical stimulation device comprising:
memory means that comprise sequences of data related to electrical pulses to be generated;
at least one electrical terminal;
control means for generating, adjusting and transmitting electrical pulses on said at least one electrical terminal according to said data sequences stored in said memory means;
**characterized in that** it comprises means for writing, in said memory means, information regarding the electrical pulses transmitted to said at least one electrical terminal, said information being settable by a user.

2. A method for the management of an electrical stimulation device, comprising the steps of:
reading, by way of control means, the content of said memory means that comprise sequences of data related to electrical pulses to be generated;
generating, regulating and transmitting electrical pulses on at least one electrical terminal according to said sequences of data related to electrical pulses to be generated, stored in said memory means;
storing in said memory means information related to the electrical pulses transmitted to said at least one electrical terminal, said information being settable by a user.

3. The method according to claim 2, **characterized in that** each one of said data sequences constitutes a work program that comprises a plurality of work steps.

4. The method according to claim 3, **characterized in that** said memory means comprise a first memory and a second memory, said data sequences related to electrical pulses to be generated comprising information for generating said electrical pulses, said first memory comprising said information for generating said electrical pulses, said second memory comprising an index to the content of said first memory and said information related to the electrical pulses transmitted to said at least one electrical terminal.

5. The method according to claim 4, **characterized in that** each element of said index comprises a reference to a particular program stored in said first memory and a space for storing said information related to the electrical pulses transmitted to said at least one electrical terminal.

6. The method according to any one of the preceding claims, **characterized in that** said information related to the electrical pulses transmitted to said at least one electrical terminal are selected from the group that comprises the intensity, repetition rate and duration of the electrical pulses transmitted in each one of said work steps.

7. The method according to any one of the preceding claims, **characterized in that** said reading step can be selected by a user and comprises a step for loading data into a temporary memory contained in said control means, said data comprising said information related to the transmitted electrical pulses.

8. The method according to claims 3 and 7, further comprising the step of overwriting, in said temporary memory, information related to the electrical pulses transmitted at the end of each work step.

9. The method according to claims 3 and 7, further comprising the step of overwriting, in said temporary memory, information related to the electrical pulses transmitted during each work step.

10. The method according to claim 3, comprising a step for managing the information related to the electrical pulses transmitted to said at least one electrical terminal and stored in said memory means, said management step comprising said read step, said read step comprising a step for loading in a temporary memory information related to the electrical pulses transmitted during the execution of a work program in a previous electrical stimulation session, said writing step comprising a step of overwriting said memory means with new information related to the electrical pulses transmitted during the execution of the same work program in the current electrical stimulation session.

11. The method according to claim 8, 9 or 10, **characterized in that** it further comprises a step for processing said information related to the transmitted electrical pulses, said processing step preceding said step of overwriting in said temporary memory and/or said step of overwriting said memory means.

12. The method according to claim 10, comprising a step for the automatic management of the information related to the electrical pulses transmitted to said at least one electrical terminal and stored in said memory means, said automatic management step comprising a second step for loading into said temporary memory information related to the electrical pulses transmitted during the execution of a work program in a preceding electrical stimulation session and an optional step for leaving said automatic management step and entering said management step following a variation of said information related to the electrical pulses transmitted during the execution of said automatic management step, said variation being performed manually by said user.

13. The method according to claim 3, further comprising, at the beginning of each one of said work steps, a step for automatically varying the values of the electrical pulses that correspond to the information stored in said memory means.

14. The method according to claim 13, **characterized in that** said automatic variation step comprises an attenuation step followed by a step for gradually restoring the values of the electrical pulses that correspond to the information stored in said memory means.

15. The method according to claim 3, **characterized in that** the duration of the work steps is set by a timer that is internal to said control means.
